# EUROPEAN PATENT APPLICATION

(11) **EP 4 458 271 A1**
(43) Date of publication of application: **06.11.2024**
(21) Application number: 23740310.0
(22) Date of filing: 12.01.2023
(51) Int. Cl.: A61B 8/00

(54) **PUNCTURE NEEDLE**

(30) Priority: 17.01.2022 JP 2022005325
(71) Applicant: Terumo Kabushiki Kaisha, Tokyo 151-0072 (JP)
(72) Inventor: FUJIKI, Jo, Ashigarakami-gun, Kanagawa 259-0151 (JP); KAMBARA, Kayo, Ashigarakami-gun, Kanagawa 259-0151 (JP)
(74) Representative: TBK
(86) International application number: PCT/JP2023/000657
(87) International publication number: WO 2023/136302

(57) **Abstract**

A puncture needle with high echo visibility of a needle tip is provided. The puncture needle includes a main body having a rod shape, a pyramidal portion that has a pyramidal shape and is disposed at a distal end of the main body, and an ultrasound reflecting structure three-dimensionally formed on a side portion of the pyramidal portion.

## Description

### Technical Field

The present disclosure relates to a puncture needle.

### Background Art

JP 2011-125632 A (Patent Literature 1) discloses an ultrasound-guided puncture needle for performing puncture while detecting a position using reflection of ultrasound and an indwelling needle having the same. This puncture needle has groove portions reflecting ultrasound on an outer peripheral surface. The groove portions include a first groove portion provided in a portion of the outer peripheral surface on the back side of a blade surface, and a second groove portion provided in a portion of the outer peripheral surface in the vicinity of a distal portion where the blade surface is formed. A plurality of the first groove portions, each of which extends in the circumferential direction and has both ends facing the blade surface, are provided in the axial direction of the puncture needle.

### Citation List

### Patent Literature

Patent Literature 1: JP 2011-125632 A

### Summary of Invention

### Technical Problem

At the time of puncture under an echo, it is important to accurately grasp a position of a needle tip in order to improve the accuracy of puncture in a procedure.

As puncture needles (see, for example, Patent Literature 1) in the related art, a puncture needle obtained by performing processing (groove processing, dimple processing, or blast processing) for ultrasound reflection on an outer peripheral surface of a body portion is exemplified. However, in such s puncture needle in the related art, there is a case where it is impossible to accurately grasp a position of a needle tip because the processing for ultrasound reflection is performed at a position different from the needle tip that is originally desired to be visually recognized. Therefore, regarding the puncture needle, it is desirable to enhance echo visibility of the needle tip.

Meanwhile, in the puncture needles in the related art as described in Patent Literature 1, there is a puncture needle subjected to processing for ultrasound reflection up to a position that is close to a distal end of a blade surface and faces the blade surface. However, there may be concerns that puncture performance of the puncture needle may be deteriorated in a case where a groove facing the blade surface is formed as the processing for ultrasound reflection, that is, in a case where the processing is performed even on a blade portion that cuts and opens the tissue. Therefore, there is also a need to achieve both the puncture performance and the echo visibility in enhancing the echo visibility of the needle tip.

The present disclosure has been made in view of such circumstances, and an object thereof is to provide a puncture needle with high echo visibility of a needle tip.

### Solution to Problem

To achieve the above object, a puncture needle according to the present disclosure includes:
a main body having a rod shape;
a pyramidal portion that has a pyramidal shape and is disposed at a distal end of the main body; and
an ultrasound reflecting structure three-dimensionally formed on a side portion of the pyramidal portion.

Further, in the puncture needle according to the present disclosure,
the pyramidal portion may have a polygonal pyramid shape having a plurality of flat portions in the side surface.

Further, in the puncture needle according to the present disclosure,
the ultrasound reflecting structure may be formed to avoid an edge portion in the flat portion.

Further, in the puncture needle according to the present disclosure,
a surface of the flat portion may include:
a blade region in which the ultrasound reflecting structure is not formed, the blade region being disposed along the edge portion; and
a reflection region in which the ultrasound reflecting structure is formed, the reflection region being disposed more inward than the blade region.

Further, in the puncture needle according to the present disclosure,
the pyramidal portion may include the flat portion in which the ultrasound reflecting structure is formed and the flat portion in which the ultrasound reflecting structure is not formed.

Further, in the puncture needle according to the present disclosure,
the pyramidal portion may have a triangular pyramid shape.

Further, in the puncture needle according to the present disclosure,
the ultrasound reflecting structure may be disposed on a distal side in the pyramidal portion.

### Advantageous Effects of Invention

According to the present disclosure, it is possible to provide the puncture needle with the high echo visibility of the needle tip.

### Brief Description of Drawings

Fig. 1 is a perspective view of a puncture needle according to the present embodiment.
Fig. 2 is a side view of the puncture needle according to the present embodiment.
Fig. 3 is a top view of the puncture needle according to the present embodiment.
Fig. 4 is a view for describing a mode of reflection of ultrasound due to an echo.
Fig. 5 is a side view of a puncture needle according to Modification 1 of the present embodiment.
Fig. 6 is a side view of a puncture needle according to Modification 2 of the present embodiment.
Fig. 7 is a side view of a puncture needle according to Modification 3 of the present embodiment.
Fig. 8 is a side view of a puncture needle according to Modification 4 of the present embodiment.
Fig. 9 is a side view of a puncture needle according to Modification 5 of the present embodiment.
Fig. 10 is a cross-sectional view taken along line X-X of Fig. 9.
Fig. 11 is a top view of another puncture needle.
Fig. 12 is a top view of still another puncture needle.

### Description of Embodiments

A puncture needle according to an embodiment of the present disclosure is described with reference to the drawings.

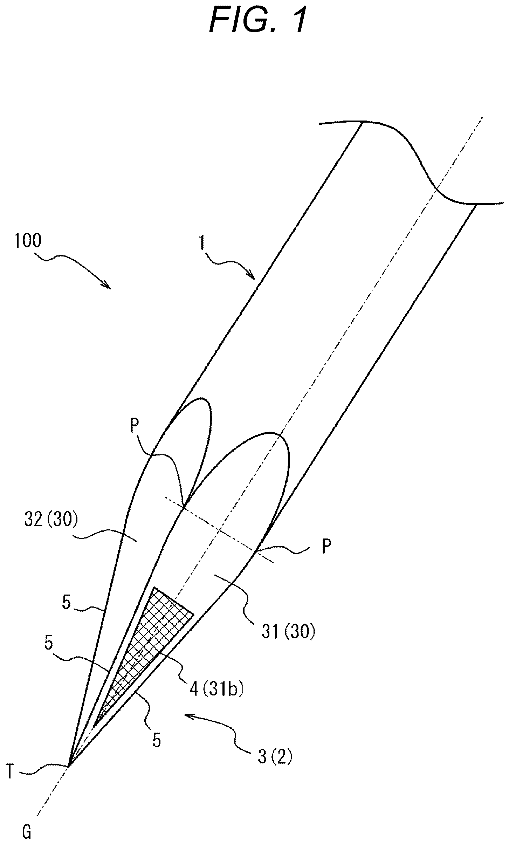

Figs. 1 to 3 illustrate a puncture needle 100 according to the present embodiment. The puncture needle 100 includes a main body 1 having a rod shape, a pyramidal portion 2 that has a pyramidal shape and is disposed at a distal end of the main body 1, and an ultrasound reflecting structure 4 three-dimensionally formed on a side portion 3 of the pyramidal portion 2.

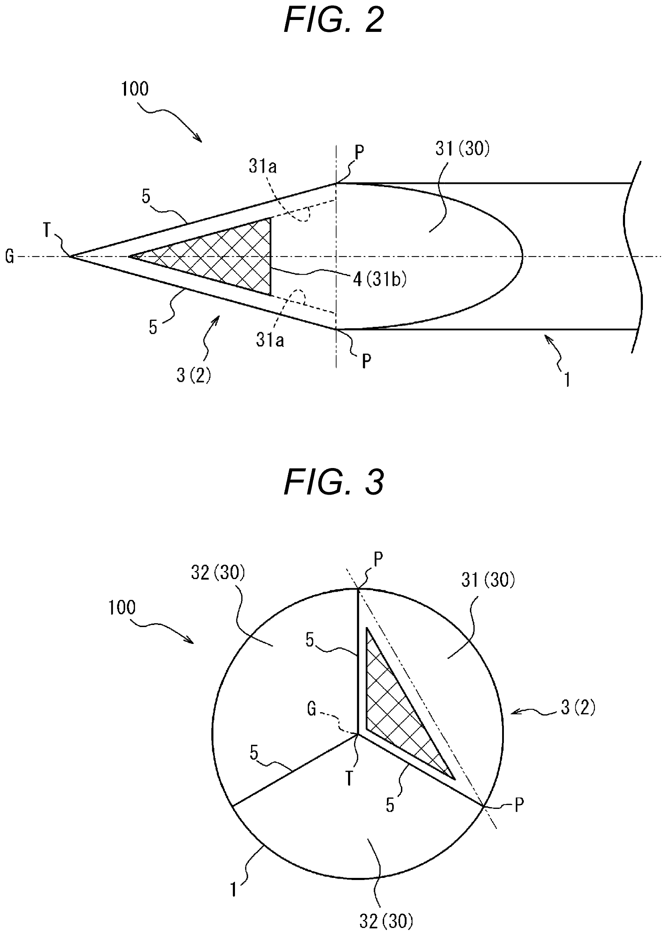

Fig. 1 is a perspective view of side surfaces of a main body 1 and a pyramidal portion 2 as viewed from a distal side of a puncture needle 100 in an oblique direction with respect to an axis G of the main body 1. Fig. 2 is a side view of the puncture needle 100 as viewed from an outer peripheral surface of the main body 1, that is, from a direction orthogonal to the axis G. Fig. 3 is a top view of the puncture needle 100 as viewed from the distal side in a direction along the axis G.

As illustrated in Figs. 1 to 3, the main body 1 has, for example, a cylindrical rod shape. The main body 1 can be made of, for example, a metal alloy such as stainless steel, a titanium alloy, or a cobalt-chromium alloy, or a resin such as Teflon or nylon. In the main body 1, a space continuously formed along the axis G may be formed inside the main body 1. That is, the main body 1 may be formed in a tubular shape that is closed on the distal side.

As described above, the pyramidal portion 2 is disposed at the distal end of the main body 1 and has a pyramidal shape. An example of the pyramidal shape is a conical shape or a polygonal pyramid shape including a plurality of flat portions on a side surface. Examples of the polygonal pyramid shape include a triangular pyramid shape, a quadrangular pyramid shape, and a pentagonal or more pyramid shape. Figs. 1 to 3 illustrate a case where the pyramidal portion 2 has a triangular pyramid shape. That is, the pyramidal portion 2 in the present embodiment has three flat portions 30 on the side surface thereof.

The pyramidal portion 2 having a polygonal pyramid shape such as a triangular pyramid can be formed by, for example, cutting or polishing a distal end of a rod-shaped material, which is to serve as the main body 1, to form flat surfaces portions which is to serve as the flat portions 30 of the polygonal pyramid. Corners of the side surface of the pyramidal portion 2, that is, boundary portions between adjacent flat portions 30 and 30 of the side surface in the polygonal pyramid (the triangular pyramid in the present embodiment) shape form blades 5 extending from a vertex T (a distal end of the puncture needle 100) toward a side portion of the main body 1.

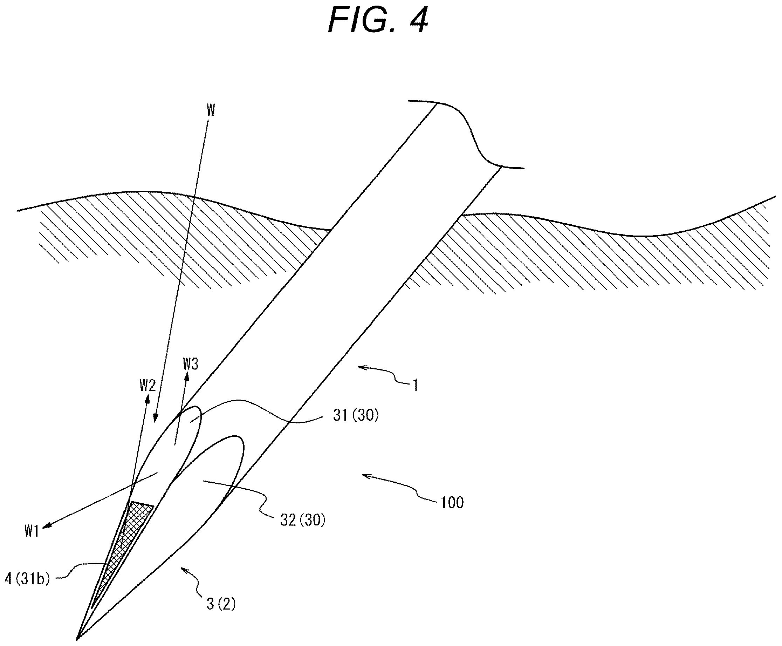

The side portion 3 is a side surface portion of the pyramidal portion 2. In the present embodiment, the side portion 3 has the three flat portions 30 as described above. The flat portions 30 include a first flat portion 31 on which the ultrasound reflecting structure 4 is formed and a second flat portion 32 on which the ultrasound reflecting structure 4 is not formed. As illustrated in Fig. 3, the flat portions 30 include one first flat portion 31 and two second flat portions 32 in the present embodiment. Note that the ultrasound reflecting structure 4 is a structure that changes a direction and an intensity of reflection of emitted ultrasound W (see Fig. 4) as will be described later.

As illustrated in Figs. 1 to 3, the flat portion 30 in the case of being viewed in a direction perpendicular to the surface thereof includes a triangular region located on the distal side and a region that has a partial elliptical or parabolic shape and is located on a rear end side of the triangular region in the present embodiment. In Figs. 1 to 3 and the like, points, located at a boundary between the triangular region and the region having the partial elliptical or parabolic shape in an outer peripheral shape of the flat portion 30, are illustrated as boundary points P and P.

The ultrasound reflecting structure 4 is formed in a partial region of the first flat portion 31. As illustrated in Fig. 2, the ultrasound reflecting structure 4, that is, the surface of the first flat portion 31 of the puncture needle 100 in the present embodiment includes a blade region 31a in which the ultrasound reflecting structure 4 is not formed and a reflection region 31b in which the ultrasound reflecting structure 4 is formed.

For example, the reflection region 31b (the ultrasound reflecting structure 4) is preferably disposed on the distal side of a line connecting the boundary points P and P. That is, the reflection region 31b (the ultrasound reflecting structure 4) is preferably disposed on the distal side of the puncture needle 100 in the pyramidal portion 2. As a result, echo visibility of a distal portion of the puncture needle 100 is improved as will be described later. Note that it is not excluded that the reflection region 31b is further provided in the flat portion 30 on a proximal side of the line connecting the boundary points P and P.

In addition, the reflection region 31b (the ultrasound reflecting structure 4) is preferably formed to avoid the vicinity of the blade 5, which is an edge portion (peripheral edge), in the first flat portion 31. That is, the blade region 31a preferably extends continuously at least along the blade 5. In addition, the reflection region 31b is preferably disposed more inward than the blade region 31a in the first flat portion 31. As a result, it is possible to maintain the sharpness of the blade 5 at the time of puncture with the puncture needle 100 and ensure favorable puncture performance. Note that all regions (including the blade region 31a) other than the reflection region 31b in the first flat portion 31 are formed in a flat surface shape in the present embodiment.

The ultrasound reflecting structure 4 is formed in a three-dimensional shape such as a groove shape, a dimple shape, and an uneven shape such as a rough surface formed by blasting. It is preferable that the ultrasound reflecting structure 4 is formed of recesses such as dents, depressions, or grooves as the three-dimensional shape such as an uneven shape. It is preferable that a surface of the ultrasound reflecting structure 4 does not protrude more than the other flat surface portion of the first flat portion 31. As a result, it is possible to avoid the ultrasound reflecting structure 4 from causing generation of resistance such as being caught by living tissue at the time of puncture, and to maintain the puncture performance. Note that a formation method or a processing method of the ultrasound reflecting structure 4 is not limited, and laser processing, cutting, rolling, pressing, or the like can be employed. The present embodiment illustrates a case where the ultrasound reflecting structure 4 is a rough surface. The rough surface of the ultrasound reflecting structure 4 in the present embodiment can be, for example, a rough surface formed by recesses obtained by performing blast processing on the reflection region 31b of the first flat portion 31 to form minute recesses and protrusions, and then removing protrusions protruding more than a flat surface of the first flat portion 31 by polishing or the like.

Due to the three-dimensional shape thereof, the ultrasound reflecting structure 4 can reflect the incident ultrasound W as a reflected wave W2 having a high intensity to some extent in a direction different from a direction of a reflected wave W1 reflected at the highest intensity by a region of the flat portion 30 where the ultrasound reflecting structure 4 is not formed, that is, the region other than the reflection region 31b as illustrated in Fig. 4.

As an example, the ultrasound reflecting structure 4 can reflect the reflected wave W2 in a direction along the incident direction of the ultrasound W at an intensity stronger than that of a reflected wave W3 which is a reflected wave of the incident ultrasound W, reflected in a direction along the incident direction of the ultrasound W from the region of the flat portion 30 where the ultrasound reflecting structure 4 is not formed. That is, since the ultrasound reflecting structure 4 is disposed in the first flat portion 31 as the flat portion 30, as illustrated in Fig. 4, when a procedure such as puncture is performed through echo observation (that is, under an echo) from the proximal side of the puncture needle 100 (a body surface side of a living body punctured with the puncture needle 100), the echo visibility is improved, whereby a position of a needle tip can be accurately grasped by the echo.

### (Description of Modifications)

Hereinafter, other preferable structures and layouts of the ultrasound reflecting structure 4 described in the above-described embodiment and variations of these structures and layouts will be described.

### (Modification 1)

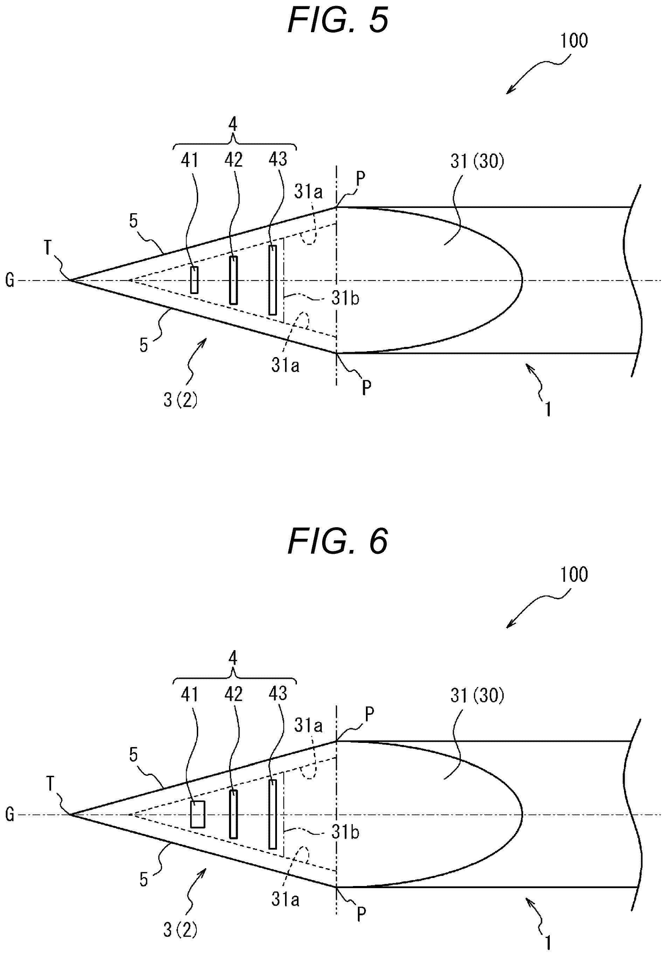

Fig. 5 illustrates a case where, as the ultrasound reflecting structure 4, transverse grooves 41, 42, and 43, which are recesses formed in the direction orthogonal to the axis G on the first flat portion 31, are formed in the reflection region 31b. The transverse grooves 41, 42, and 43 are disposed in this order from the distal side at equal intervals, for example, along the axis G. Lengths of the transverse grooves 41, 42, and 43 in a direction intersecting the axis G increase in the order of the transverse grooves 41, 42, and 43. Since the transverse grooves 41, 42, and 43 are formed in the direction orthogonal to the axis G, ultrasound, emitted as an echo from the body surface side of the living body punctured with the puncture needle 100, is reflected to the body surface side at a strong intensity when the procedure such as puncture is performed through the echo observation from the proximal side of the puncture needle 100, so that the echo visibility is improved, whereby the position of the needle tip can be accurately grasped by the echo.

### (Modification 2)

Fig. 6 illustrates a case where the transverse groove 41 in the ultrasound reflecting structure 4 illustrated in Modification 1 is formed to be deeper than the other transverse grooves 42 and 43. As a result, echo visibility of a most distal portion of the puncture needle 100 is improved.

### (Modification 3)

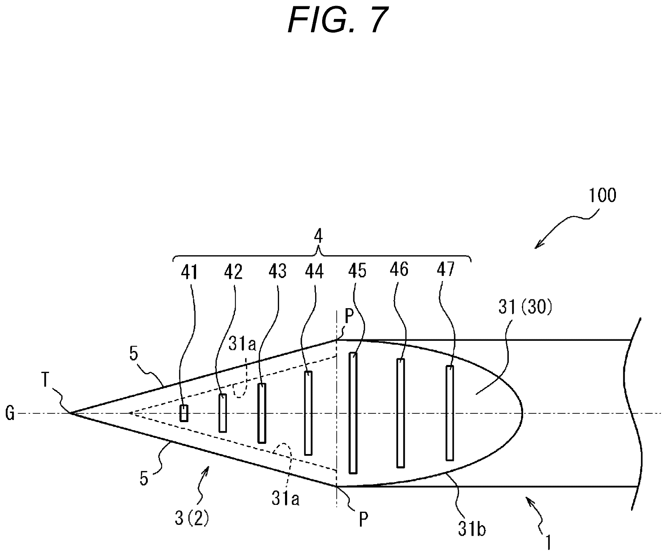

Fig. 7 illustrates a case where, as the ultrasound reflecting structure 4, transverse grooves 41 to 47, which are recesses formed in the direction orthogonal to the axis G, are formed in the reflection region 31b using portions of the surface of the first flat portion 31 on both the distal side and the proximal side with respect to the line connecting the boundary points P and P, except for the blade region 31a, as the reflection region 31b. The transverse grooves 41 to 47 are disposed from a distal portion to a proximal portion of the first flat portion 31 along the axis G. A length in the direction intersecting the axis G increases in the order of the transverse grooves 41, 42, 43, 44, and 45. In addition, the transverse grooves 45,46, and 47 become shorter in this order. Since the ultrasound reflecting structure 4 is formed widely over the entire surface of the first flat portion 31 in this manner, the overall echo visibility of the first flat portion 31 is improved, whereby the position of the needle tip can be accurately grasped by the echo.

### (Modification 4)

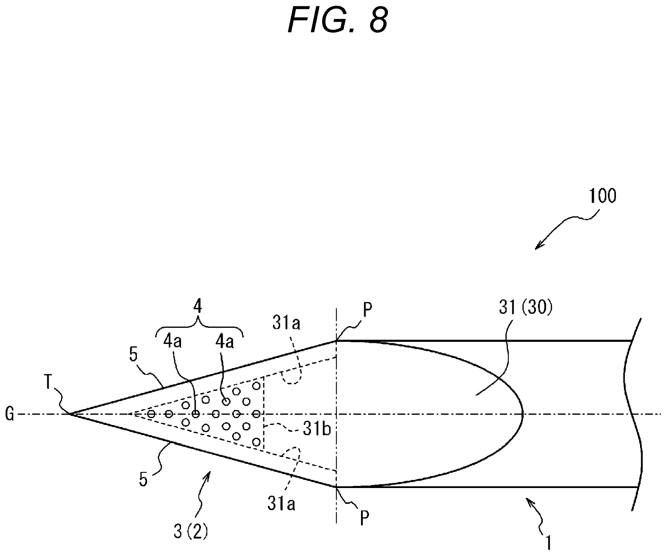

Fig. 8 illustrates a case where, as the ultrasound reflecting structure 4, round recesses 4a each having a dimple shape are densely formed in the reflection region 31b. Even if a shape of the ultrasound reflecting structure 4 is modified in this manner, the echo visibility is improved as in Modification 1 and the like, whereby the position of the needle tip can be accurately grasped by the echo.

### (Modification 5)

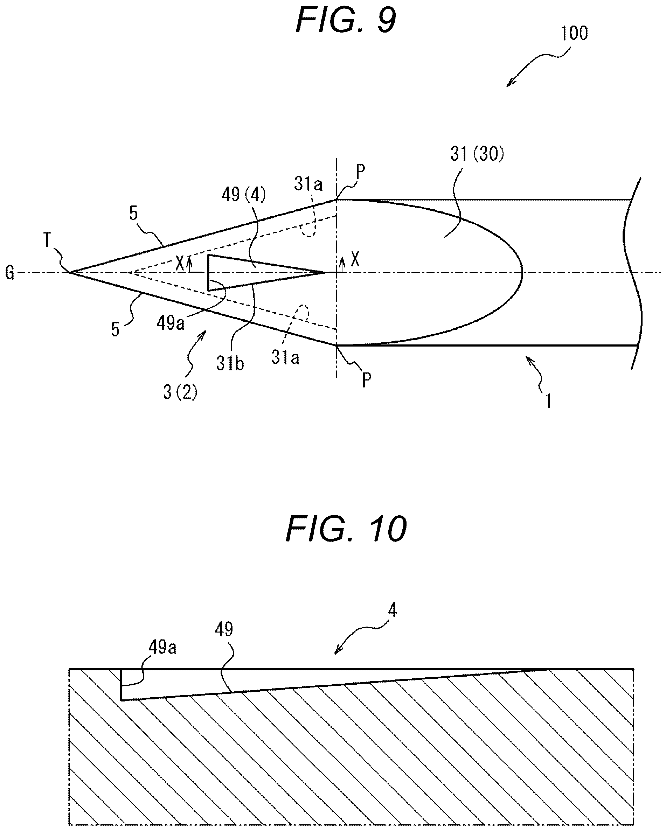

Fig. 9 illustrates a case where, as the ultrasound reflecting structure 4, a triangular groove 49, which is a triangular recess having a base on the distal side and a top on the proximal side, is formed in the reflection region 31b when viewed from above perpendicularly to the surface of the first flat portion 31. Fig. 10 is a cross-sectional view taken along line X-X in Fig. 9. As illustrated in Figs. 9 and 10, the triangular groove 49 is formed such that a depth of the recess sequentially increases from the top to the base side, that is, from the proximal side to the distal side of the puncture needle 100.

On the base side (distal side) of the triangular groove 49, a groove wall surface 49a descending toward a bottom of the groove is formed. The groove wall surface 49a faces the proximal side in the direction along the axis G in the top view of the puncture needle 100. When such a groove wall surface 49a is formed, it is possible to reflect the ultrasound W (see Fig. 4) incident from the proximal side by the groove wall surface 49a such that the reflected wave W2 is reflected at a strong intensity in the direction along the incident direction of the ultrasound W (see Fig. 4) when the procedure such as puncture is performed through the echo observation from the proximal side of the puncture needle 100. Therefore, the echo visibility is improved, whereby the position of the needle tip can be accurately grasped by the echo.

In addition, since the triangular groove 49 is formed such that the depth of the recess sequentially increases from the proximal side to the distal side of the puncture needle 100, a bottom surface of the triangular groove 49 hardly interferes with the reflected wave W2 (see Fig. 4) from the groove wall surface 49a. As a result, the echo visibility is improved.

For example, it is preferable that the groove wall surface 49a extends from an edge of an upper end of the groove of the triangular groove 49 so as to be orthogonal to the first flat portion 31 (see Fig. 9). As a result, the reflected wave W2 (see Fig. 4) can be reflected at a stronger intensity in the direction along the incident direction of the ultrasound W (see Fig. 4).

As described above, it is possible to provide the puncture needle that achieves both the puncture performance and the echo visibility.

### [Other Embodiments]

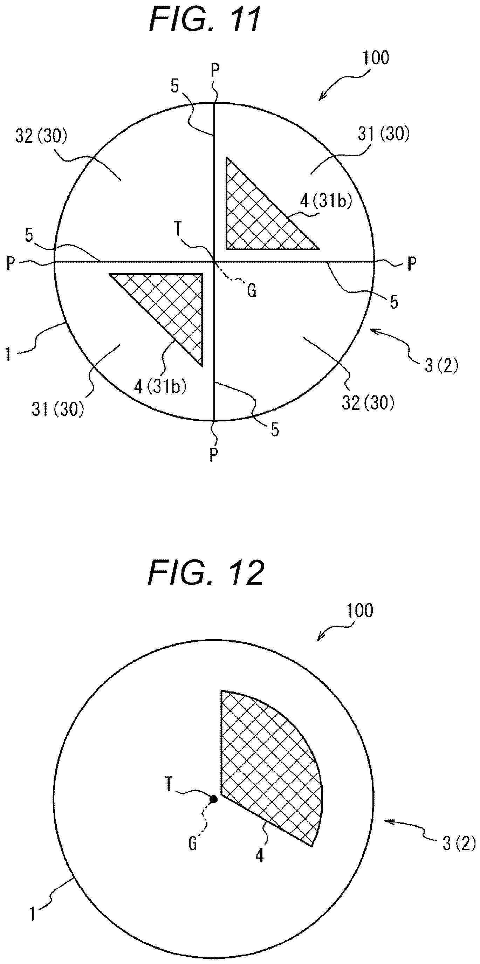

(1) The case where the pyramidal portion 2 has the triangular pyramid shape has been described as an example in the above-described embodiment. However, the pyramidal portion 2 is not limited to the triangular pyramid shape as described above. Fig. 11 illustrates a case where the pyramidal portion 2 of the puncture needle 100 is a quadrangular pyramid.
(2) In the above-described embodiment, the description has been made regarding the case where the pyramidal portion 2 of the puncture needle 100 has the triangular pyramid shape, the side portion 3 has the three flat portions 30, the flat portions 30 include the first flat portion 31 in which the ultrasound reflecting structure 4 is formed and the second flat portion 32 in which the ultrasound reflecting structure 4 is not formed, and the flat portions 30 include, for example, the single first flat portion 31 and the two second flat portions 32. However, the flat portions 30 may include first flat portions 31 more than the second flat portion 32 (for example, may include two first flat portions 31 and one second flat portion 32). Since a portion where the sound wave reflecting structure 4 is formed and a portion where the sound wave reflecting structure 4 is not formed are present in the side portion 3 of the pyramidal portion 2 in this manner, it is possible to grasp a position of the puncture needle 100 in the circumferential direction (circumferential direction with the axis G as the center) by the echo as necessary. Note that a case where all the flat portions 30 are the first flat portions 31 is not excluded from the case where the pyramidal portion 2 has a polygonal pyramid shape such as a triangular pyramid shape in the present embodiment.
(3) In the above-described embodiment, the description has been made regarding the case where the pyramidal portion 2 of the puncture needle 100 has the triangular pyramid shape, the side portion 3 has the three flat portions 30, the flat portions 30 include the first flat portion 31 in which the ultrasound reflecting structure 4 is formed and the second flat portion 32 in which the ultrasound reflecting structure 4 is not formed, and the flat portions 30 include, for example, the single first flat portion 31 and the two second flat portions 32. However, the pyramidal portion 2 may have a polygonal pyramid shape such as a quadrangular pyramid shape as described above. Fig. 11 illustrates a case where the pyramidal portion 2 has a quadrangular pyramid shape. Note that Fig. 11 is a top view of the puncture needle 100 in which the pyramidal portion 2 has the quadrangular pyramid shape as viewed from the distal side along the axis G. Fig. 11 illustrates a case where the first flat portion 31 and the second flat portion 32 are alternately disposed along the circumferential direction on the side portion 3. Since the first flat portion 31 and the second flat portion 32 are alternately disposed in this manner, it is possible to more favorably grasp the position of the puncture needle 100 in the circumferential direction by the echo.
(4) Although the case where the pyramidal portion 2 of the puncture needle 100 has a polygonal pyramid shape has been described as an example in the above-described embodiment, the pyramidal portion 2 may have a conical shape as illustrated in Fig. 12. In this case, the ultrasound reflecting structure 4 may be formed on a part or the whole of the side portion 3 of the pyramidal portion 2. Fig. 12 illustrates a case where the ultrasound reflecting structure 4 is formed on a part of the side portion 3 of the pyramidal portion 2.

Note that the configurations disclosed in the above-described embodiments (including the other embodiments, the same applies hereinafter) can be applied in combination with configurations disclosed in other embodiments as long as there is no contradiction, and the embodiments disclosed in the present specification are examples, and the embodiments of the present disclosure are not limited thereto, and can be appropriately modified within a scope not departing from the object of the present disclosure.

### Industrial Applicability

The present disclosure is applicable to a puncture needle.

### Reference Signs List

- 1: Main body
- 100: Puncture needle
- 2: Pyramidal portion
- 3: Side portion
- 30: Flat portion
- 31: First flat portion
- 31a: Blade region
- 31b: Reflection region
- 32: Second flat portion
- 4: Sound wave reflecting structure
- 4a: Round recess
- 41: Transverse groove
- 42: Transverse groove
- 43: Transverse groove
- 44: Transverse groove
- 45: Transverse groove
- 46: Transverse groove
- 47: Transverse groove
- 49: Triangular groove
- 5: Blade
- G: Axis
- P: Boundary point
- T: Vertex
- W: Ultrasound
- W1: Reflected wave
- W2: Reflected wave
- W3: Reflected wave

## Claims

1. A puncture needle comprising:
a main body having a rod shape;
a pyramidal portion that has a pyramidal shape and is disposed at a distal end of the main body; and
an ultrasound reflecting structure three-dimensionally formed on a side portion of the pyramidal portion.

2. The puncture needle according to claim 1, wherein the pyramidal portion has a polygonal pyramid shape having a plurality of flat portions in the side surface.

3. The puncture needle according to claim 2, wherein the ultrasound reflecting structure is formed to avoid an edge portion in the flat portion.

4. The puncture needle according to claim 3, wherein
a surface of the flat portion includes:
a blade region in which the ultrasound reflecting structure is not formed, the blade region being disposed along the edge portion; and
a reflection region in which the ultrasound reflecting structure is formed, the reflection region being disposed more inward than the blade region.

5. The puncture needle according to any one of claims 2 to 4, wherein the pyramidal portion includes the flat portion in which the ultrasound reflecting structure is formed and the flat portion in which the ultrasound reflecting structure is not formed.

6. The puncture needle according to any one of claims 2 to 5, wherein the pyramidal portion has a triangular pyramid shape.

7. The puncture needle according to any one of claims 1 to 6, wherein the ultrasound reflecting structure is disposed on a distal side in the pyramidal portion.
